(19) Europäisches Patentamt
European Patent Office
Office européen des brevets

(11) **EP 4 344 650 A1**

(12) **EUROPEAN PATENT APPLICATION**

(43) Date of publication:
**03.04.2024 Bulletin 2024/14**

(21) Application number: **23152170.9**

(22) Date of filing: **18.01.2023**

(51) International Patent Classification (IPC):
**A61B 8/06** (2006.01)     **A61B 8/12** (2006.01)
**A61B 8/08** (2006.01)     **A61B 5/00** (2006.01)
**A61B 6/00** (2024.01)

(52) Cooperative Patent Classification (CPC):
**A61B 8/06; A61B 5/0066; A61B 6/481;**
**A61B 6/482; A61B 6/5247; A61B 8/0891;**
**A61B 8/12; A61B 8/5223; A61B 8/5261**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **30.09.2022 US 202263411820 P**

(71) Applicant: **Koninklijke Philips N.V.**
**5656 AG Eindhoven (NL)**

(72) Inventors:
• **HAASE, Hans Christian**
**Eindhoven (NL)**
• **GRASS, Michael**
**Eindhoven (NL)**

(74) Representative: **Philips Intellectual Property &**
**Standards**
**High Tech Campus 52**
**5656 AG Eindhoven (NL)**

(54) **IVUS ENABLED CONTRAST AGENT IMAGING WITH DUAL ENERGY X-RAY**

(57)     System (SYS) and related method for image processing, comprising an input interface (IN) to receive input data comprising i) spectral projection imagery of a region of interest including a conduit (CN) for passage of a liquid (CA), the spectral projection imagery obtainable in an imaging procedure by a spectral X-ray imagining apparatus (IA1) with contrast agent (CA) present in the liquid, and ii) additional image data acquirable by a further imaging apparatus (IA2) of the non-ionizing type, the said additional image data representative of 3D information of the conduit. A predictor component (PC) predicts based on the spectral projection imagery and the additional image data, a concentration of contrast agent in the said conduit.

FIG. 1

**Description**

FIELD OF THE INVENTION

The invention relates to system for image processing, to related methods, to an imaging arrangement, to a computer program element, and to a computer readable medium.

BACKGROUND OF THE INVENTION

Spectral X-ray systems, like a C-arm system with a dual layer detector for example, enable more advanced imaging methods, in particular, but not only, during minimally invasive procedures. Spectral imaging allows imaging for particular materials.
When imaging for structures of poor radio-opacity, such as blood vessels, for example cardiac coronaries, contrast agent is used to achieve better image contrast in respect of those structures.
Contrast agent quantification, blood to contrast dilution measurement, and the dynamic behavior of contrast agent are measures that can help extract quantitative information of blood flow from X-ray images. For example, blood flow volume, velocity, and distribution have broad physiological significance for vascular diseases like coronary artery disease and peripheral artery disease, etc. Such flow-related diagnostic quantities/measures may be used to diagnose disease, plan procedures, evaluate outcomes, etc. Use of spectral imaging system, such as dual energy X-ray alone already provides improved options for contrast agent-based analysis of X-ray images.
However, in some cases even more precision in relation to such diagnostic quantities/measures may be called for.

SUMMARY OF THE INVENTION

There may therefore be a need for improved contrast agent-based imaging with improved utility, in particular with enhanced extraction of physiological information. In particular, extraction of reliable and accurate physiological information may be needed.
An object of the present invention is achieved by the subject matter of the independent claims where further embodiments are incorporated in the dependent claims. It should be noted that the following described aspects of the invention equally apply to the related method, to the imaging arrangement, to the computer program element and to the computer readable medium.
According to a first aspect of the invention there is provided a system for image processing, comprising:

at least one input interface configured to receive input data comprising i) spectral projection imagery of a region of interest including a conduit for passage of a liquid, the spectral projection imagery obtainable in an imaging procedure by a spectral X-ray imagining apparatus with contrast agent present in the liquid and ii) additional image data acquirable by a further imaging apparatus of the non-ionizing type, the said additional image data representative of 3D information of the conduit; and
a predictor component configured to predict, when the system is in use, based on the spectral projection imagery and the additional image data, at least a concentration of contrast agent in the said conduit.
In embodiments, the 3D information is presentative of a lumen of the conduit.

In embodiments, the system further comprising a flow assessment component configured to compute a quantity of interest based at least on the predicted concentration of contrast agent in the said conduit, the quantity of interest descriptive of one or more flow characteristics of the liquid.
In embodiments, the said quantity of interest is a medical quantity of interest.
In embodiments, the said medical quantity of interest includes any one of more of: volumetric flow, propagation time of contrast agent through at least a section of said conduit, liquid-contrast agent mixing behavior, and transluminal attenuation gradient. The flow may relate to collateral flow.
Thus, system affords a more cost-effective and more practicable way (in particular, but not only, for interventions) to estimate such quantities, previously out of reach without expensive rotational X-ray based CT scanner tomographic equipment which is not required herein.
In embodiments, the input data includes in-situ pressure readings, and wherein the quantity of interest computable by the flow assessment component includes a pressure estimate for a distal end of a section of the conduit.
In embodiments, the predictor component or the flow assessment component is configured to compute an initial concentration of the contrast agent upon entering the conduit and/or a rate of entering into the conduit of the said contrast agent.

System of any one of the previous claims, wherein the conduit is part of a vascular, urinary or lymphatic system of mammal, such as a human patient.

In embodiments, the further imaging apparatus is configured for intra-vascular imaging.

System of any one of the previous claims, wherein the further imaging apparatus is any one or more of: i) an ultrasound imaging apparatus or ii) an optical tomographic coherence imaging apparatus.

In embodiments, the spectral X-ray imagining apparatus is of the C-arm type, having a detector configured for energy discriminative detection of X-ray intensities.

In embodiments, the spectral projection imagery includes a times series of frames.

In embodiments, the spectral projection imagery includes a coronary angiogram.

In embodiments, the computed concentration of contrast agent and/or the quantity of interest is provided for processing, wherein the processing includes any one or more of: i) displaying on a display device an indication of the concentration of contrast agent and/or of the quantity of interest, ii) storing same in a medical data storage, and iii) controlling operation of medical equipment, iv) processing by decision support system.

In embodiments, the predictor component based on a machine learning model.

In another aspect there is provided an imaging arrangement comprising the system of any one of the previous claims, further including the spectral X-ray imagining apparatus.

In embodiments, the arrangement may include the second imaging apparatus. The second imaging apparatus need to be configured for spectral imaging, but may well be so in some embodiments, if required.

In another aspect there is provided a method of image processing, comprising:

receiving input data comprising i) spectral projection imagery of a region of interest including a conduit for passage of a liquid, the spectral projection imagery obtainable in an imaging procedure by a spectral X-ray imagining apparatus with contrast agent present in the liquid, and ii) additional image data acquirable by a further imaging apparatus of the non-ionizing type, the said additional image data representative of 3D information of the conduit; and

predicting, when the system is in use, based on the spectral projection imagery and the additional image data, at least a concentration of contrast agent in the said conduit.

In another aspect there is provided a method of training the ML model based on training data.

In another aspect there is provided a computer program element, which, when being executed by at least one processing unit, is adapted to cause the processing unit to perform the image processing method or the ML training method.

In another aspect there is provided at least one computer readable medium having stored thereon the program element, or having stored thereon the machine learning model.

It is proposed herein to combine the spectral (such as dual energy) X-ray projection image data with additionally sourced 3D lumen information in respect of the conduit, such as by using intravascular ultrasound (IVUS) data, OTC data, or other additional non-ionizing imaging modality to enable accurate dilution quantification and bolus propagation tacking, etc. The conduit CN may be a blood vessel (such as coronary arteries or vein, or other), and the liquid/fluid may be blood including an admixture of contrast agent in any concentration.

Without the 3D lumen information from, preferably, intravascular imaging the blood with contrast agent dilution can only be derived under assumption of various simplifications, e.g., homogeneous background, circular vessel shapes, no foreshortening etc. Whilst such modelling for obtaining the 3D lumen information without additional imaging is not necessarily excluded herein, in preferred embodiments such additional imaging is indeed used as this allows a more accurate, patient-bespoke estimation of blood flow volume. Uncertainties in flow velocities estimated from the bolus propagation can be reduced herein.

The combination of 3D IVUS vessel data and spectral (such as dual energy) X-ray projection image data, preferably collected at various locations of a vascular structure, has been found to lead to good results. This combination allows a particularly accurate assessment of blood-to-contrast agent dilution/concentration. Furthermore, bolus propagation speed and volumetric blood flow rates are estimated. The collection of such 3D vessel data and of spectral (such as dual energy) X-ray projection image data at plural locations in the vessel/conduit may also be done for other additional imaging modalities other than IVUS, such as OTC or others still. The second imaging modality is preferably interventional, but not necessarily so.

Thus, what is proposed herein is the combined processing of spectral X-ray projection data with additionally sourced 3D information in respect of the conduit system of interest. The proposed approach harnesses the supreme contrast agent focused imaging capability of spectral projection imaging, and additional 3D lumen information that can be sourced with the described non-ionizing imaging modalities (such as IVUS or OTC) safer and computationally cheaper than by using CT X-ray based reconstructions.

Such 3D information sourced from, in particular, IVUS or OTC, is also more accurate than CT (computed tomography) scanning, and does not need to be explicitly registered computationally to the 2D spectral images since, both image streams are recorded in the same session. With intravascular imaging, the 2D spectral and the 3D information is preferably recorded in parallel at the same time thus providing native registry of the two image streams.

The X-ray projection images may be supplied by a dual layer C-arm system that delivers spectral intraprocedural images. Other spectral setups such as dual source, photon-counting, etc, may also be used.

As used herein, the said conduit is preferable and moistly a part of vasculature (an artery or vein) but in some applications the conduit may a part of contrast agent delivery equipment, such as portion of a catheter or tube through whish contrast agent is released into the vasculature or other body part of interest. At least a part of the catheter/tube, such as its tip portion, is recorded in the spectral projection imagery and the principles described herein can be used by system/method to compute/determine in particular an initial contrast agent concentration as mentioned above. This is preferably done by using prior knowledge on the shape of the relevant catheter/tube and/or its material composition. If the (preferably) 3D shape is known, and most cases it is, the initial concentration may be determined in this manner even without using the 3D information from the second imaging apparatus. Thus, if the only the initial concentration is required, no second imaging apparatus is needed.

"*user*" relates to a person, such as medical personnel or other, operating the imaging apparatus or overseeing the imaging procedure. In other words, the user is in general not the patient.

In general, the term "*machine learning*" includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of training data corpus is called "training". In general task performance by the ML module may improve measurably, with training experience. Training experience may include suitable training data and exposure of the model to such data. Task performance may improve the better the data represents the task to be learned. "*Training experience helps improve performance if the training data well represents a distribution of examples over which the final system performance is measured*". The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 at 1.2.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Fig. 1 show a block diagram of a medical imaging arrangement as may be used in particular to support contrast-based imaging;
Fig. 2 shows a schematic drawing of a conduit such as a vessel in which contrast agent is residing therein;
Fig. 3 shows a block diagram of a computerized system for computing a contrast agent concentration;
Fig. 4 shows a schematic representation of a graphics display according to one embodiment envisaged herein;
Fig. 5 shows a block diagram of a machine learning model as may be used herein in embodiments of the system of Fig. 1;
Fig. 6 shows a training system for training a machine learning model;
Fig. 7 shows a flow chart of a method of computing contrast agent concentration based on spectral projection imagery and the use of such computed contrast agent concentration; and
Fig. 8 shows a method of training a machine learning model.

DETAILED DESCRIPTION OF EMBODIMENTS

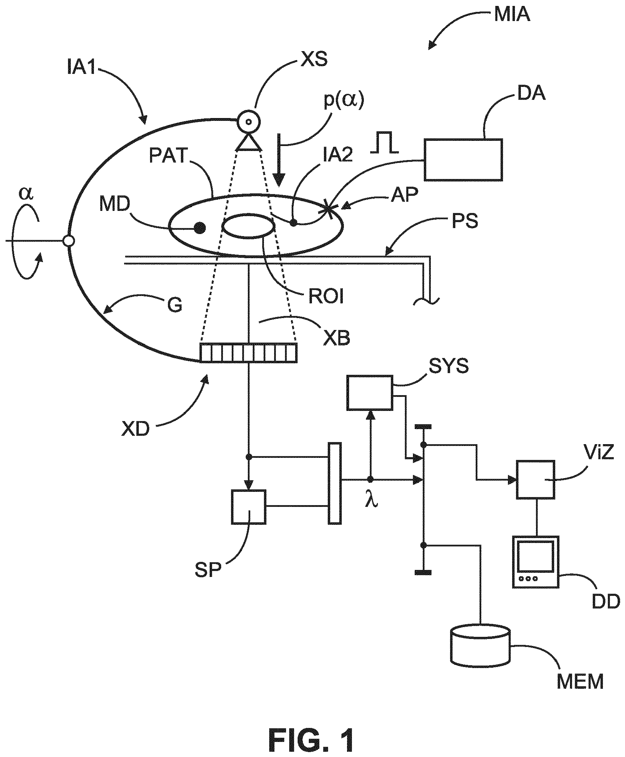

Referring first to Fig. 1, this provides a block diagram of a medical imaging arrangement MIA configured to facilitate computing flow related quantities, which may be used to assess a blood vessel lesion in a patient for example. Specifically, and as will be explained in more detail below, the computer-implemented system SYS is configured herein for accurate computation of a concentration of a contrast agent in a liquid. The liquid with the contrast agent passes through a conduit CN, such as pipe or tube system or similar. The contrast agent concentration may then be used as input to compute other diagnostic quantities of interest that relate to flow characteristics of a liquid with the contrast agent as it passes through the conduit CN. Blood flow through vessels CN, such as arterial or veinal coronaries in the human heart, or through other blood vessels of other organs are mainly envisaged herein. However, other conduit structures such as lymphatic systems, urinary, gastric, etc., are not excluded herein, and neither are non-medical applications such as examinations in speleology or hydrology, examinations of inaccessible plumbing systems, of hydraulic system or of other pipework in machine parts in vehicles for example, or in any other type of machinery, etc.

The medical imaging arrangement MIA may include a, preferably, X-ray based imaging apparatus IA (also referred to herein simply as "imager"). The imaging apparatus IA may be of the C-arm/U-arm type so allows collection of projection imagery $\lambda$ along different projection directions $P(\alpha)$ around the region of interest, such as around the mentioned stricture in a coronary vessel. The imaging apparatus IA includes an X-ray source XS and an X-ray sensitive detector XD. In some embodiments, the X-ray source XS, and opposite thereto, the X-ray sensitive detector XD, are arranged preferably on a rotatable gantry G. The gantry G may have a recess in which an examination region is defined where the patient, or in particular the region of interest, resides during imaging. The rotatable gantry may thus have a "C", "U" or similar shape, such as in C-arm imaging apparatus envisaged herein. However, such or similar rotational or an otherwise dynamic setup, where there is at least some relative motion between source and detector intended, is not necessarily required herein in all embodiments. Instead, a planar radiographic setup is also envisaged in some embodiments. Instead of the rotatable setup as illustrated in Fig. 1, a bi-planar imaging setup with two detectors with intersecting imaging axes (for example at right angles) may be used.

In addition, whilst digital-at-source imaging, such as flat panel detectors is preferred, where the imagery is natively acquired as digital data, such as setup is not necessarily required. More traditional, cassette-operable, analog imaging methods (based on X-ray films) are not excluded herein. The analog imagery may be digitized post-acquisition for example, by using a digital camera. Imaging based on X-ray image intensifiers, etc with post-digitization is also envisaged in some embodiments.

Generally, imaging includes energizing the X-ray source XS, so that an X-ray beam XB issues forth from a focal spot of the source XS, traverses the examination region (with the region of interest in it) and interacts with patient tissue matter. Interaction of X-ray beam XB with tissue matter causes the beam to be modified. The modified beam may then be detected at the detector XD. Conversion circuitry (not shown) converts detected intensities into digital imagery, in particular into projection imagery. The imagery may be used by user in the medical procedure. The patient may reside on a patient support PS such as a table during imaging. Soft tissue such as the one making up the coronary vessels, provide usually poor contrast in native X-ray imagery. In order to boost contrast, a contrast agent (sometimes "CA" or short), such as an iodine solution, gadolinium, or other, is delivered through an access point AP into the blood flow of the patient prior to or during imaging. The contrast agent CA may be delivered via a CA delivery apparatus DA, such as a motorized pump, but manual delivery is not excluded herein. The delivery apparatus DA may be operable to deliver a defined volume of contrast agent to the patient. The volume of contrast agent so delivered travels with the blood flow. After a certain period of time contrast agent accumulates at the region of interest such as in or around the stricture. Image acquisition is preferably synchronized with arrival of contrast agent at the ROI. Preferably "contrasted" imagery is acquired whilst there is contrast agent CA present at the ROI. Optionally, additional "non-contrasted" imagery is acquired whilst no contrast agent is present at the ROI.

The, in particular contrasted, imagery may be acquired in a time series with varying concentration of the contrast agent accumulating at the region of interest. Image acquisition may start before, may continue throughout, and may terminate after accumulation of the contrast agent at the ROI. In general, contrast agent concentration increases over time after delivery, will plateau at a certain maximum value, and will then wash out or decrease over time until completely vanished. Whilst a single or certain discrete number of still images may be acquired in some instances, acquisition of a stream images, or frames, acquired at a suitable frame rate is preferred as this allows real time dynamic observation. Specifically, the acquired stream of projection imagery may be visualized by a visualizer VIZ as a live video feed on display device DD. Alternatively or in addition, still imagery is so visualized. Operation of the facilitator system SYS is preferably based on processing such still or stream imagery. The imagery may be stored in an image memory MEM or may be processed otherwise.

X-ray imaging is generally based on the attenuation co-efficient of the matter(s) that makes up the region of interest. The attenuation (coefficient) is dependent on the energy of the X-ray beam. In order to harness this energy dependency, a spectral imager IA may be used, configured for spectral imaging. This may include detector-sided or source-sided solutions. Detector-sided solutions include photon-counting detector hardware or a dual-energy setup with dual-layer hardware for example. More than two detector layers may be used although this is less common. Source-side solutions include multi-source setups, kVp switching, filtration and other.

The imaging set-up in spectral imaging allows acquisition of multi-dimensional image data. Such multi-dimensional image data includes projection imagery at different energy levels, instead of acquiring energy integrated imagery as provided by a conventional (non-spectral) X-ray imaging set-up, the latter not being excluded herein. For example, such a conventional set-up may be used for digital angiography subtraction ("DAS") where pairs of projection images at higher and lower concentration of the contrast agent is acquired. Such image pairs are then spatially registered and subtracted from each other to boost contrast of the soft tissue including the contrast agent. DSA may also be practiced with spectral imaging setup.

Preferably however, for more accurate quantification of the contrast agent concentration/volume at the region of interest, a spectral set-up is preferred. Such a spectral imaging setup includes a spectral image processor SP. The spectral image processor SP may implement a spectral image processing algorithm, such as material decomposition or other. A range of such spectral image processing algorithms are envisaged herein. The spectral processor SP processes the multi-energy projection imagery λ acquired by the source- or detector-sided imaging set-up just described, and computes therefrom spectral imagery that differs from the original input multi-energy projection imagery. Spectral imagery is thus computationally derived from the input multi-energy projection imagery. Such spectral imagery, which may be in projection domain, may include for example, a monochromatic image at a desired energy level, or a virtual non-contrast image, or a contrast-only image (e.g. iodine image), a Compton scatter image, and others. The monochromatic image may correspond to the attenuation co-efficient of the contrast agent used. For example, the energy value or energy range for the monochromatic image may be chosen to equal or include the K-edge energy of the particular contrast agent to be used for good contrast for example. However, such as K-edge based choice is not mandatory herein and other energy values or ranges for the mono-chromatic imagery is equally envisaged herein. The monochromatic image represents a computational approximation of an image that may have been obtained had a monochromatic X-ray source XS at the respective energy level been used for imaging. In most practical cases, the actual X-ray source XS is polychromatic. The virtual non contrast ("VNC") image represents an approximation of an image that may have been obtained if there had been no contrast agent present in the field of view of the imager IA, at the exclusion of other materials. The contrast ("VC") image represents an approximation of an image that may have been obtained if there had been only contrast agent present in the field of view of the imager IA, at the exclusion of other materials. Thus, image contrast in VC is exclusively or pre-dominantly contrast agent focused. It is preferably the spectral (projection imagery) so computed by spectral processor SP that is processed by the facilitator system SYS, instead or, or in addition, to the original multi-energy input projection imagery, as will be described below in more detail.

For simplicity, the designation "λ" may be used herein to indicate input imagery in general to be processed by the facilitator system SYS. That is, "λ" may relate to original projection imagery as acquired or, preferably, to the spectral projection imagery as computed by the spectral processor SP based on the original projection imagery as acquired. The spectral processor SP and the facilitator system SYS may be implemented on different computing systems, possibly geographically separated such as in a distributed "cloud" architecture. However, implementation on a single computing system is also envisaged herein.

Imaging as envisaged herein is preferably attenuation-based, but this is not at the exclusion of other X-ray modalities, such as dark-field imaging and/or phase contrast imaging. Whilst tomosynthetic or tomographic imaging is not excluded herein, due to the multi-directional imaging capability of the preferably rotational imaging apparatus IA, processing of projection imagery solely in projection domain is mainly envisaged herein in embodiments, in addition to sourced 3D information that is generally computationally cheaper to procure than tomographic reconstructions as will be explored on more detail below. No tomographic reconstruction algorithm is required herein which saves computation time. The spectral processor SP may thus operate in projection domain.

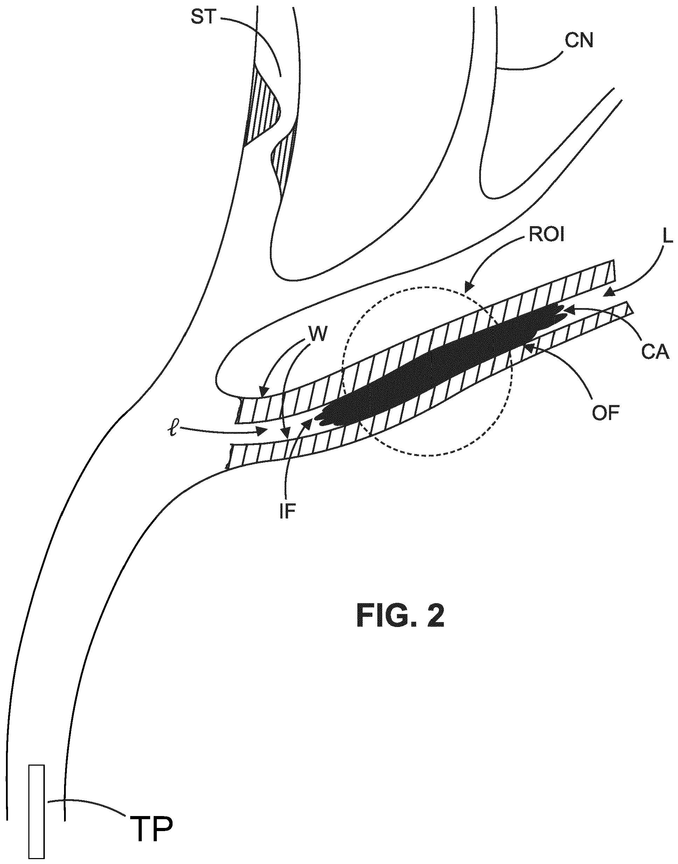

Reference is now made to Fig. 2 which shows a schematic representation of a situation that may be encountered when imaging conduits CN through which liquid with contract agents CA passes and as envisaged herein in embodiments. In preferred embodiments, the conduit (system) CN may include an arterial or a veinal vessel system preferably of the human heart or of other organs. As indicated in the upper branch of Fig. 2, a stenosis may narrow lumen L of the vessel system. Such stenosis may be due to a build of deposits such as plaque. Such stenosis may lead to health problems. The correct identification of such a stenosis, its severity, or other aspects thereof, can be done by an image-based examination the flow characteristics of the blood in the affected vessel CN. Mixing into the blood of contrast agent confers better imaging contrast of the lumen's spatial structure as the blood-contrast agent admixture passes through the vessel CN and fills the said lumen.

With continued reference to Fig. 2, this affords a sectional view of such a vessel system including a branch through which contrast agent CA is passing with the blood flow along the length $\ell$ of the respective branch. A region of interest ROI "shown in dashed lines may be geometrically defined either automatically (eg, by segmentation) or user prescribed (by using a pointer tool for example). The region of interest may be demarked by a graphical user interface on the acquired imagery and/or may be done by collimator action as required. The contrast agent concentration computations as described herein may be related to the said region of interest. Thus, a main interest herein is the computation of contrast agent CA concentration in the part of the vessel CN as defined by the ROI. The ROI may relate to a subpart of the vessel system CN such as shown in Fig. 2, such as to one or more branches (with or without its collaterals), or may relate to the whole imaged vascular system in the current field of view. Thus, the contrast agent concentration refers to the amount, such number of particles, volume or mass of contrast agent in a reference volume as defined by the region of interest in the respective vessel CN. In particular, it is amount of contrast agent in the lumen L that is of main interest. Lumen L represents the (whole) space inside the vessel CN surrounded by vessel wall W. Preferably it is the volumetric concentration or mass concentration of contrast agent CA in respect of the section of the lumen L inside ROI that may be computed herein. For example, mass concentration may be defined as the ratio of mass of contrast agent by the lumen volume L in the ROI considered. The contrast agent concentration may be essentially zero, when there is only blood present in the ROI, such as prior arrival of the administered amount of contrast agent (bolus) as it travels with the blood flow. At the other extreme, the concentration may be such that there is substantially no blood present, such as at time when contrast agent displaced all blood in the ROI considered. The contrast agent concentration will be understood to vary over time. Thus, contrast agent concentration may relate to the current concentration at a given instant/frame, or may relate to an average of contrast agent concentration readings/values over time, or to another such consolidated quantity that combines contrast agent concentration information.

Although other applications of the principles described herein in medical and non-medical applications in support of image-based examination of conduit systems are also envisaged herein, we will be referring herein mainly to the conduit CN as a blood vessel CN, with the understanding that the said other applications are not excluded herein. Also, the contrast agent CA will be referred to herein at times as iodine, although it will be understood that other radiation-opaque substances such as gadolinium, a mixture thereof, or other substances can also be used herein. Whilst X-ray imaging is mainly contemplated herein, other imaging modalities such as MRI or others still are not excluded, in which case the contrast agent acts may have suitable properties than to increase radio-opacity in order to suitably confer better image contrast. Fig. 2 also illustrates in its lower left a projection footprint of a tip portion TP of a catheter or tube through which administration contrast occurs, and as may appear in the FOV of some frames if required.

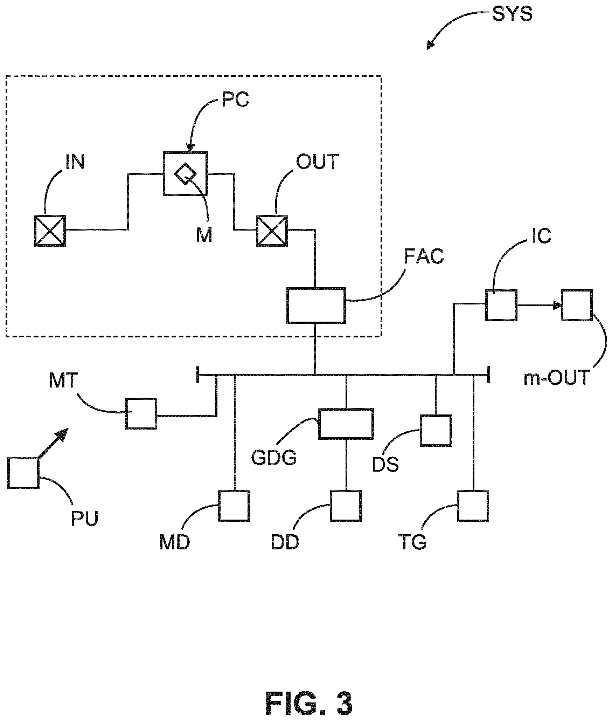

Reference is now made to the block diagram of Fig. 3 which shows components of the system SYS for facilitating improved contrast agent-based imaging, Specifically, the system may facilitate determining contrast agent concentration in a vessel CA or section thereof. The system may further facilitate determining one more blood flow related diagnostic quantities based on said contrast agent concentration. The system SYS may be configured for at least two-channel processing. Specifically, the system may include one or more input ports IN through which at least two-channel input data is received. The two-channel input data includes as one channel a single or a stream of angiographic spectral X-ray imagery acquired by the spectral imager IA1, and, as the other at least one channel, spatial 3D information/data.

The spatial 3D information describes the 3D volumetric structure or configuration of the vessel CN, in particular its lumen L, at the ROI, whilst the spectral X-ray imagery represents quantity or amount of contrast agent residing at a given time in the vessel CN at the region of interest considered. The specifically, contrast in the spectral X-ray imagery may be modulated by the mass of the contrast agent present in the ROI. Thus, the spectral X-ray imagery may represent mass of contrast agent in the vessel section CN at the ROI.

In some embodiments, the input data and the system is three-channel. Thus, the input data includes in addition to spatio-structural lumen 3D data and spectral imagery, in-situ pressure readings collected by pressure reading device MD.

The two- or three-channel input data is processed jointly by the system SYS to compute the contrast agent concentration in the vessel CN at the ROI. Specifically, such multi-channel input data is processed by a predictor component PC of system SYS to predict such concentration. example, in mass per volume, for example in milligrams of contrast agent per milliliter or in other suitable quantity. The output data provided by predictor component PC at output interface OUT represents the concentration of contrast agent currently residing in the region of interest considered.

Thus, the system proposed herein combines information from at least two channels, from the (angiographic) spectral X-ray imagery stream, and from the additional 3D information in order to produce an accurate estimate of the contrast agent concentration.

The input data, in particular the spectral X-ray imagery, may include a time series of frames ("stream"). In such cases, the output data provided by the predictor component PC may include correspondingly a time series of such concentration values/readings. Alternatively, multiple frames are proceeds herein to compute a single contrast agent concentration value as output. Operation of the system SYS is preferably in real-time and/or dynamic. Specifically, new/updated concentration readings are computed by the predictor component PC as new frames are received at input data. The 3D lumen information as per the second channel may be updated accordingly or may be used for one or more spectral angiographic frames as required. The single concentration readings or the series of such concentration readings are output at output interface OUT. We will simply refer herein as concentration values for brevity, it being understood that such concentration values are of the contrast agent CA considered herein. Also, it will be understood that the output data may include a single such concentration reading for a given frame, or may include a time series of concentration values, one or more for each frame considered and received at input port IN.

The concentration values as provided via output interface OUT may be of interest in their own rights and so may be stored, processed, or displayed on a display device DD as required.

A monitoring device MT may be used for monitoring the computed concentration values. The monitoring device MT may use thresholding. If the concentration value exceeds, or, as the case may be, falls short of such threshold, an alert signal may be issued to alert the user. The alert signal may be visual, audio, haptic or a combination of any one of the foregoing.

However, in general it is quantities derivable from the concentration values that are of interest herein. Thus, a flow assessment component FAC may be used to compute, based on the output one or more concentration values, related flow characteristics of the liquid comprising blood and the contrast agent. Such flow characteristics of the blood/contrast agent mixture based on contrast agent concentration may be used beneficially to inform diagnostic or therapeutic steps downstream. Diagnostic quantities may be computed by the flow assessment component FAC based on the one or more concentration values. The flow characteristics or diagnostic quantities may be stored or processed.

For example, the diagnostic quantities may be passed on to a diagnostic system DS to provide diagnostic information, such as a score for the presence of a medical condition. The scores and/or diagnostic quantities may be processed by a graphics display generator GDG to generate a graphics display for display on display device DD. The graphics display may include a representation of the diagnostic one or more quantities. The flow related diagnostic quantities may be displayed in combination with the concentration values and/or the score in the graphics display on the display device DD.

The monitoring device MT (or a different such device) may be used to monitor the said quantities derived from the concentration values, instead of or in addition to, the concentration values themselves.

The graphics display generated by generator GDG may further include at least a part of the input imagery, in addition to the output data such as the concentration values and/or the flow characteristics derived therefrom.

The computed diagnostic quantities and/or concentration values may be supplied to a control interface CI to control other types of medical equipment. The control interface CI provides control signal $m\text{-}OUT$ to control said equipment based on the computed diagnostic quantities and/or concentration values. For example, the control signal $m\text{-} m\text{-}OUT$, may be provided as a feedback system to control stetting of one or both of the imagers IA2 IA2, of the measurement device (such as in-situ pressure measurement device), etc. A tagger TG may be optionally used to tag respective frame and/ optionally the associated 3D measurement d with the respective, computed contrast agent concentration C.

The structural spatial 3D information that describes in particular the configuration of the lumen in the region of interest in the vessel CN considered preferably derived or includes imagery supplied by the second imaging modality/imager IA2. The second imaging modality IA2 may be configured to supply such 3D structural imagery of the lumen. A suitable such 3D imaging modality IA2 includes ultrasound ("US") imaging or optical coherence tomography ("OCT") imagery. Preferably, the second imaging modality IA2 is of the non-ionizing type to reduce health risks to the patient. Again, single such 3D imagery, such as US or (intravascular) OTC imagery, may be supplied and processed by system SYS as a time series/stream, preferably in timewise registry with the spectral imagery still frame or frames. Processing of a single shot frame is not excluded herein however.

Preferably, the second imaging modality IA2 for providing the 3D lumen structural information it is of the invasive type, such as IVUS. In IVUS, a suitably miniaturized US imaging probe is introduced through the access point AP into the patient and delivery to the ROI through a catheter. The US imaging probe may include an US transducer to generate in-situ (within the vessel CN) sound waves which interact with the wall structure of the lumen at the region of interest. Reflections thereof are registered by counterpart US receiver and processed into ultrasound imagery to reveal lumen 3D structures. The US transducer and US receiver a preferably integrated into the probe as a transceiver component, although such transceiver arrangement is not a necessity herein. Suitable US technologies may include PMUTs or CMUTs. Either one can be designed for multi-frequency IVUS applications. In such technologies, one or more vibrating elements ("active source"), configurable as a piezo-resistor or a capacitive membrane, transmit and receive different carrier frequencies. In many systems, a discrete set or array of such transducer elements, each tunable to a certain frequency.

Similarly, and also invasively, using a suitable light probe, optical imagery may be used in OTC to obtain the 3D structure information. Other types of such interventional imaging modalities may include endoscopic ultrasound imaging, where the probe is introduced down the patient's esophageal tract to be positioned proximal the heart.

However useful such interventional imaging is where a suitable probe is introduced into the patient (in particular, but not necessarily, into the vessel CN), external imaging may be used instead at times such as ultrasound imaging applied from outside. In addition still, the spatial 3D information provided as input data alongside the spectral X-ray projection imagery, may not necessarily include imagery acquired during the intervention, but may instead pertain to prior imagery obtained in prior imaging session(s), such as MRI, CT, CTA or others. Such prior imagery for a given patient may be retrieved for the given patient from a medical database and can be processed herein instead of, or in addition to current interventional imagery as supplied by the second imaging modality IA2.

Preferably, interventional imaging is used such as the described IVUS system or OTC with the angiographic data streams to combine them suitably as described herein for processing by the predictor component PC to yield accurate estimation of concentration of contrast agent. IN particular, the combination of IVUS imagery and angiographic spectral data have been found to have particular synergy in producing accurate and realistic contrast agent concentration estimates.

The predictor component PC may be arranged herein as a machine learning model M suitably trained on training data as will be described in more detail below. However, modelling other than by machine learning, such as via traditional analytical methods based for example of thresholding and/or segmentation and anatomical and physical understanding of the conduit CN and/or flow properties of the liquid of interest are also considered herein. Hybrid approaches are also considered where part of the computation is based on machine learning whilst the others use said analytical methods/modelling.

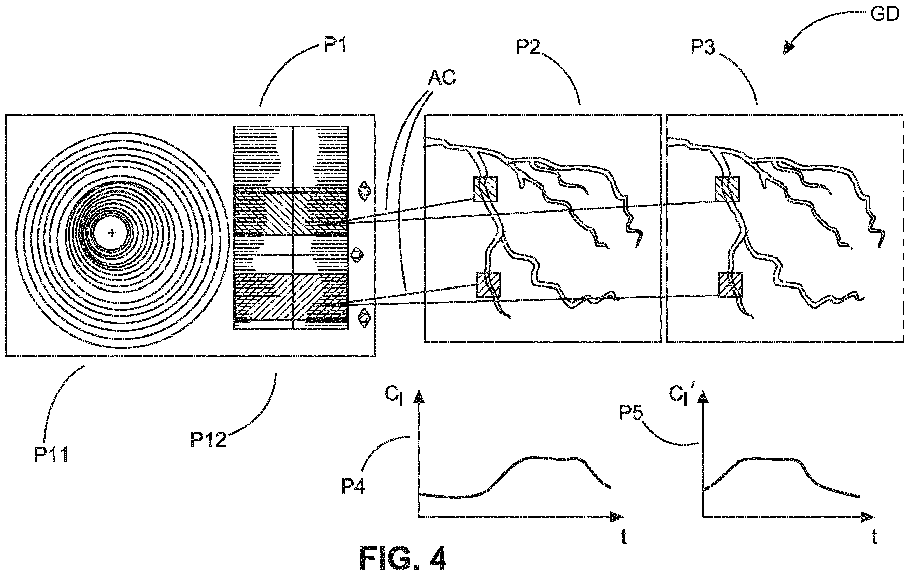

Reference is now made to Fig. 4 which shows a schematic view of a graphics display GD generatable by graphics display generator GDG, based on the output data. The graphics display GD may be displayed on the display device DD during the intervention or thereafter in a post-analysis session for example.

The graphics display GD according to embodiments may include multiple panels Pj. The multi-panel graphics display GD may include a panel P1 which represents the interventional image data, in particular the 3D structural information of the lumen. Fig. 4 illustrates IVUS as an embodiment in multiple views, for example, along the lumen's length $\ell$, and/or in plan-view, reconstructable from the UV measurements taken inside the lumen.

One or more additional panels P2, P3 (a single such additional panel may suffice in some embodiments) include the angiographic spectral image frames. Again, only one frame may be displayed in single panel P2 or P3, alongside and concurrently with the panel P1 for the 3D image information. Panels P1, P2, P3 may be displayed concurrently, or user can on request cycle through them in sequence.

Panel P1 may include two or more sub-panels such as P11, P12 as illustrated. One such sub-panel P11 may include imagery taken along the length of the lumen, whilst the one or more other sub-panel P12 includes plan view(s) thereon.

Preferably one or more graphical associator components AC may be used to support user in spatially associating 3D information in panel P1, with angiographic projection data information in panels P2 and/or P3. For example, image portion(s) in the 3D image panel P1 may be highlighted by overlay structures in different colors, for example, which are associated by lines or other associative symbology widgets with corresponding locations in the projection imagery in panel P2, P3.

Optionally, one or more curves $C_I$, $C_{I'}$ are shown which represent concentration values collected over the time as shown in panels P4, P5, corresponding to spectral projection data in one or more panels P2, P3. Again, a single panel with a single projection image may be sufficient, optionally associated with a second panel, such as P4, that shows the associated filling curve for this projection data.

The filling curves $C_I$, $C_{I'}$ illustrate the typical phases as expected whilst the concentration agent bolus passes through the associated region of interest. There is a ramp up phase where concentration increases, followed by a plateau phase where the contrast agent saturates and remains relatively constant, and a follow-up drop-off phase where the contrast agent is washed out as it egresses the region of interest.

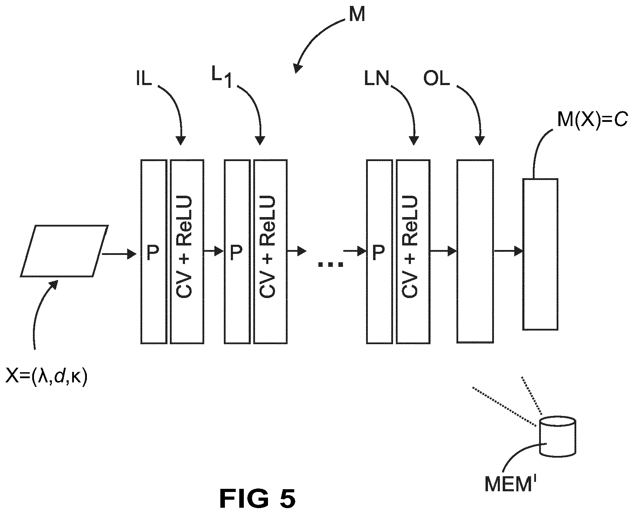

Reference is now made to Fig. 5 which shows a schematic diagram of a machine learning ("ML") model M that may be used for the implementation of the predictor component PC.

In ML approaches, a latent relationship between image-observable contrast in the spectral projection imagery in combination with the 3D information and the corresponding contrast agent concentration is learned implicitly from training data, and does not necessarily require an explicit analytic ad hoc modelling. Instead, a generic machine learning model M architecture may be used instead. Based on previous training data, as may be found in medical data bases, this relationship is implicitly modelled by adjusting certain parameters of the machine learning model based on the training set in a training phase. Once sufficiently trained (as may be determined by tests), the trained model M can then be deployed into medical practice as described above to estimate the contrast agent concentration accurately and robustly.

Additional learning input may be provided to enrich the input data (Z,d) by contextual data $\kappa$. The contextual data $\kappa$ may include some of all of the physiological patent characteristic, and/or some of the in-situ measurements. The contextual data $\kappa$ may include pre-operative 3D imagery, such as CT, MRI or other, so that the model M can take surrounding anatomy into account for better learning. Instead or in addition, contextual data $\kappa$ may include bio-characteristics such as sex, age, weight, height, etc.

Initial (for example arbitrarily chosen) parameters $\theta^k$ of the machine learning model M can be adapted in on or more iterations k, based on the training data (possibly enriched by contextual data $\kappa$) to capture this latent mapping or relationship between combo-input data ($\lambda$,d) and contrast agent concentration C. However, as mentioned above, such a machine learning model approach is not necessarily required and other, in particular analytical modelling approaches are also envisaged as they may provide results in sufficient approximation in some instances.

With continued and more detailed reference to Fig. 5, this shows a block diagram of the machine learning model M as may be used herein in some embodiments. The machine learning model may be arranged as an artificial neural network ("NN") for example, preferably as an NN of the convolutional type, referred to as "CNN". CNNs are useful in processing spatially correlated data such as image data, the processing of which is envisaged herein as explained earlier.

The network M may comprise a plurality of computational nodes arranged in layers in a cascaded fashion, with data flow proceeding from left to right and thus from layer to layer. The model may thus be of the feedforward type. Recurrent networks are not excluded herein, especially if the input data ($\lambda$,d) has time-series character, such as data stream collected over a time period. Such is specifically, envisaged herein as such time series type input data have been found to yield trained model of good robustness. However, the processing of input data ($\lambda$,d) where sone or both of the two-channels ($\lambda$ and d) comprises single frame is also envisaged herein.

The model network M may be said to have a deep architecture because it has more than one hidden layers. In a feed-forward network, the "depth" is the number of hidden layers Lj ($1 \leq j \leq N$) between input layer IL and output layer OL, whilst in recurrent networks the depth is the number of hidden layers, times the number of passes.

The machine learning model includes certain parameters, such as weights of convolution filters CV, that are adjusted in the training phase which will be explained in more detail below. Once trained, that is, once the parameters are suitably adjusted, input X, including input contrasted imagery $\lambda$ (in particular contrasted projection imagery $\lambda$) and the 3D data $d$ (such as IVUS image data), possibly enriched by contextual data $\kappa$, is input and received at an input layer IL during deployment or testing. The input X is hence either of the form (Z,d) or ($\lambda$, d, $\kappa$). The input data X in deployment or testing propagates through the layers of the model and emerges as the desired result M(X)=$C$ as the output layer OL. Thus, at output layer OL, the desired result is produced including in some embodiments the concentration $C$ in a suitable unit, such as *mg/ml,* etc. Thus the output may be a regressed into a scalar value $C$ Instead of regression, a classification may be sufficient in some embodiments where the output merely indicates into which range/interval of concentration values the concentration value C falls, such as $C \in$ [5*mg/ml,10mg/ml*] for example.

Thus, the machine learning model *M*, for example the neural network example shown in Fig. 5 or others, can be understood to act as a transformer, where the two or higher dimensional data (($\lambda.d$) or ($\lambda,d$, $\kappa$) ) is transformed into a single scalar value C, as the input data progresses through the layers IL, $L_j$($j{\geq}1$),OL of the network *M*.

The output layer OL may be a regression layer where the input data, including imagery ($\lambda.d$) or ($\lambda,d$, $\kappa$) is regressed into a single scalar value C. Alternatively, a classifier layer OL may be used, where the input is classified into *C*-value-ranges/intervals as mentioned.

In case of a classification result, the output layer OL may be configured as a *softmax*-function layer or as similar computational nodes where feature maps from previous layer(s) are combined into normalized counts to represent the classification probability or scores per class. In a regressional setup, OL may be fully connected layer.

Preferably, some or all of the hidden layers Lj ($1{\leq}j{\leq}N$), and optionally the input layer, are convolutional layers, that is, include one or more convolutional filters CV which process an input feature map from an earlier layer into layer-output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit RELU as shown, or as a *soft-max*-function, a sigmoid-function, tanh-function or any other suitable non-linear function. As opposed to a convolution layer, the output layer may be fully connected layer. Hybrid networks, including fully connected and convolutional layers are also envisaged herein.

Additional optional layers may be used in CNN model M, such as drop-out layer, pooling layers P, and other. The pooling layers reduce dimension of output whilst drop-out layer sever connections between nodes from different layers.

A hybrid model is also envisaged, optionally including a pre-processing network for example, such as an autoencoder (AE) or a variational AE (VAE). The preprocessor preprocesses the input first to produce intermediate output and it is this intermediate output that is then classified or regressed into the contrast agent concentration value C or range as described above. For example, the preprocessor AE may be trained to segment the input image for main vessels, micro vessels and perfused tissue for example. Thus, portions of the input image may be so classified first into segmentation classes. Then, for each class, the remaining network may determine contrast amount in each vessel class/concentration. Thus, in general, the preprocessor of whichever kind, AE or not, may be used to produce location dependent C(s) values (with s indicating an image location or neighborhood/subset) as opposed to a global value for the whole image. Both, localized or global C value computations are envisaged herein in embodiments.

The trained model may be stored in one or more data storages MEM' .

Preferably, to achieve good throughput, the computing device PU includes one or more processors (CPU) that support parallel computing, such as those of multi-core design. In one embodiment, GPU(s) (graphical processing units) are used.

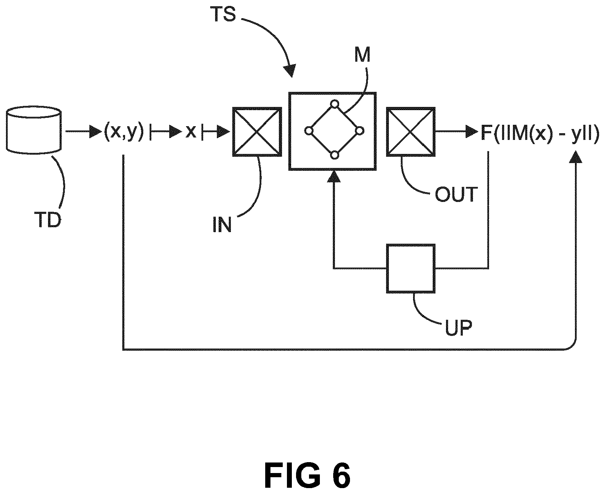

Fig. 6 shows a schematic diagram of training system TS for training the machine learning model, such as the one shown in Fig. 5 or other.

Training system TS may be used to train such a model M based on training data (*x,y*) as may be held in a training data memory TD. Training data (*x,y*) can be procured from existing medical data such as may be found in a medical data bases, such a PACS, HIS or other database or storage system. Such training data may include historic image data or other health records of patients that have undergone similar examinations in which hemodynamic indices were computed, or at least where contrasted imagery of a related ROI was acquired. The historic imagery may be reviewed by a human expert, for example. The expert may review the contrasted imagery of historic patients and may select historic imagery that corresponds, according to their medical knowledge, to a certain contrast agent concentration C. The human expert may review the patient health record for notes on the respective patient to come to their conclusion. Thus, the human expert may assign a respective score *y* (or label) to different respective samples of training input x=($\lambda.d$). y may be an estimate of the for the concentration C. Alternatively, label y may relate to a concentration range.

Respective (historic) context data $\kappa$, such as historic concentration estimates, etc may be used to assist the user when assigning their score y. The context $\kappa$ may be found in the health record of historic patients. In some cases, the health record context c may be used to infer the correct concentration value C. Specifically, medical records associated with historic imagery may be harvested either by a human user or by an automatic text analyzer tool (such as an NLP (natural language pipeline) or string matcher tool, such as *grep* or other) to extract the corresponding concentration value, as this information may be sometimes mentioned in the respective reports or records referring to a specific image frame or series of frames such as an average concentration value. In this way, the collection process can be fully or partially automated, thus allowing to procure suitably labelled training data construct more quickly.

Alternatively to searching and assessing historic training data, experiments may be conducted on a phantom to acquire suitable pairs of i) image+3D information and ii) their associated contrast concentration, where both aspects i),ii) can be varied/controlled by the experimenter. Such experimental setup may also be used to determined coefficient of an analytical model function.

Training, as administered by training system TS, is the process of adapting parameters of the model based on the training data. An explicit model is not necessarily required as in some examples it is the training data itself that constitutes the model, such as in clustering techniques or k-nearest neighbors. etc. In explicit modelling, such as in NN-based approaches and many others, the model may include as system of model functions/computational nodes, with their input and/or output it least partially interconnected. The model functions or nodes are associated with parameters $\theta$ which are adapted in training. The model functions may include the convolution operators and/or weights of the non-linear units such as a RELU, mentioned above at Fig. 5 in connection with NN-type models. In the NN-case, the parameters $\theta$ may include the weights of convolution kernels of operators CV and/or of the non-linear units.

The parameterized model may be formally written as $M^\theta$. The parameter adaptation may be implemented by a numerical optimization procedure. The optimization may be iterative. An objective function f may be used to guide or control the optimization procedure. The parameters are adapted or updated by updater UP so that the objective function is improved. The input training data x' is applied to the model. The model responds to produce training data output $M(x^i)$ = $y^i$. The objective function is mapping from parameters space into a set of numbers. The objective function f measures a combined deviation between the training data outputs y' and the respective targets $y^i$. Parameters are iteratively adjusted to that the combined deviation decreases until a user or designer pre-set stopping condition is satisfied. The objective function may use a distance measure $\| . \|$ to quantify the deviation.

In some embodiments, but not all, the combined deviation may be implemented as a sum over some or all residues based on the training data instances/pairs $(x^i, y^i)^i$, and the optimization problem in terms of the objective function may be formulated as:

$$argmin_{\,\theta}\, f = \sum_k \| M^\theta(x_k), y_k \| \qquad (1)$$

In the setup (1), the optimization is formulated as a minimization of a cost function f, but is not limiting herein, as the dual formulation of maximizing a utility function may be used instead. Summation is over training data instances i.

The cost function fmay be pixel/voxel-based, such as the L1 or L2-norm cost function. For example in least squares or similar approaches, the (squared) Euclidean-distance-type cost function in (1) may be used for the above mentioned regression task. When configuring the model as a classifier, the summation in (1) is formulated instead as one of cross-entropy or Kullback-Leiber divergence or similar. Huber cost function may be used.

The exact functional composition of the updater UP depends on the optimization procedure implemented. For example, an optimization scheme such as backward/forward propagation or other gradient based methods may be used to adapt the parameters $\theta$ of the model M so as to decrease the combined residue for all or a subset $(x_k, y_k)$ of training pairs from the full training data set. Such subsets are sometime referred to as batches, and the optimization may proceed batchwise until all of the training data set is exhausted, or until a predefined number of training data instances have been processed.

Training may be a one-off operation, or may be repeated once new training data become available.

Optionally, one or more batch normalization operators ("BN", not shown) may be used. The batch normalization operators may be integrated into the model M, for example coupled to one or more of the convolutional operator CV in a layer. BN operators allow mitigating vanishing gradient effects, the gradual reduction of gradient magnitude in the repeated forward and backward passes experienced during gradient-based learning algorithms in the learning phase of the model M The batch normalization operators BN may be used in training, but may also be used in deployment.

The training system as shown in Fig. 6 can be considered for all learning schemes, in particular supervised schemes. Unsupervised learning schemes may also be envisaged herein in alternative embodiments. GPUs may be used to implement the training system TS.

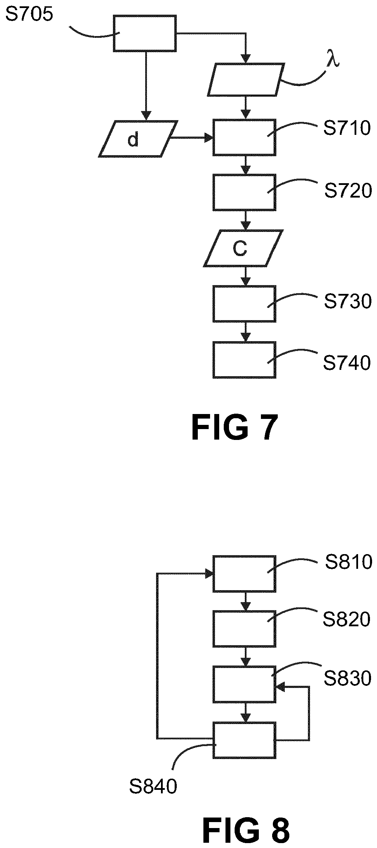

Fig. 7 shows a flow chart of a computer-implemented method that may be used to implement the above-described system SYS for the computation of contrast agent concentration and, optionally, volumetric flow quantities of blood such as diagnostic quantities derivable from the said concentration values. It will be understood however, that the below described steps are not necessarily tied to the system described above, but may be understood as teaching in their own right.

At step S705 at least 2-channel input data is obtained. This step may include obtaining spectral X-ray projection data, using spectral processing. Step S705 may further include obtaining the 3d lumen information, such as by OTC or IVUS or other. The 3D lumen information may be obtained in the IVUS embodiment in a pullback operation where the US probe is moved passed the stricture ST. Step S705 may further include a spatial or time registration of the two image data streams, the spectral projection data $\lambda$ and the 3D IVUS or OTC or other imaging modality data. The spectral projection data $\lambda$ may include for example coronary CN angiographic frames (contrast agent present) and, optionally, fluoroscopy frames (no contrast agent). Such registration step is optional, as in IVUS for example native registration can be achieved by parallel acquisition in the two channels.

In some embodiments, the initial concentration of contrast agent bolus as administered when recoding the angiographic frames may be assumed to be known or is computed herein, as will be explained in more detail below. The initial (that is, initially administered) contrast agent concentration and/or the administration rate of contrast agent may form contextual data $\kappa$ which is provided herein as additional input for the systems (in particular for model M) to be processed alongside the spectral input imagery. Such additional input may be used whether or not ML is used. Use of such contextual data allows for better results, better performance, better learning, etc.

The IVUS/OTC 3D data d and the spectral X-ray projection data can be obtained in any order or, preferably in parallel, at the same time.

At step S710 input data is received. The input data is preferably two channel as described, and includes the spectral x-ray projection data $\lambda$ and, as a second channel, 3D information d on the lumen structure of the vessel or other conduit to be examined/imaged.

Preferably, the 3D lumen information is provided by a second imaging modality IA2, in addition to the x-ray spectral imager IA1 for the first channel $\lambda$. In particular, IVUS is used in preferred embodiment as the second imaging modality IA. Alternatively, OTC may be used instead or in addition. Any other, preferably non-ionizing imaging modality, capable of providing 3D information on the lumen structure is also envisaged herein. In less preferred embodiments, modelling of the lumen may be used, based on pre-operative data. Whilst image data is preferred, this may not necessarily required, as geometrical modelling based on shape primitives, such as mesh models, may be used instead or in addition intra- or inter-operative image data or pre-operative prior image data. A generic vessel/lumen model may be used, derived from average measurements taken form a sample population of patients. In some generic models, the lumen may be set to have an assumed, idealized circular cross-section. Alternatively, the generic model may be adapted based on bio-characteristics of the given patient. Preferably however, the 3D information is image data acquired in the current procedure. The combination of angiographic spectral projection data with US, in particular IVUS, has been found to be yield good results. The input imagery $\lambda$ and the 3D information d is preferably obtained whilst some amount of contrast agent is residing at least partly in the region of interest (such as section) of the vessel structure CN of interest. The two channel input data $(\lambda, d)$ may be a time series acquired over time as the contrast agent bolus passes through the region of interest.

The input imagery $\lambda$ is preferably obtained based on projection data acquired by spectral X-ray imager IA, such as dual energy or photon counting setup. The projection data is processed by a spectral image processing algorithm to obtain the input spectral X-ray projection $\lambda$ as a contrast-agent-only-image, as mentioned above.

At step S720 the two channel input data $(\lambda, d)$ is combined/processed using a machine learning algorithm or analytical modelling to compute an estimate C for the contrast agent concentration in the region of interest.

The computed concentration C may be output at step S730 for further processing such as storages, display or for used in a control operation of medical equipment for the current exam, or for feeding into a diagnostic system, etc.

In a preferred embodiment such further data processing may include step S740 at which blood flow characterizing one or more quantities are computed, based in particular on the contrast agent concentration value as provided in step S730. The initial contrast agent as computed herein may be used in this step for some quantities if needed.

The concentration value may be a single value at a given time or preferably includes a time series of such values, such as a filling curve, where different contrast agent concentration values are computed at different time instants. The different time instants preferably correspond to time instants of the input data, in particular to the acquisition time of the spectral projection imagery and/or the acquisition time of the 3D information such as provided by the IVUS or by any other suitable 3D capable non-ionizing additional imaging modality used.

In embodiments, the input data is 3-channel, thus includes a third channel of additional in-situ (that is, in-vessel) pressure readings as may be collected at the ROI by a pressure measurement device. Such additional data may include vital signal measurements, such as heart frequency, temperature, etc. Yet in addition, the further data received over the third channel may further include contextual information $\kappa$ that describes bio-characteristics of the patient. This may be used for analytical modelling but more preferably for machine modelling. Use of such context data allows making the machine learning procedure more efficient and/or making the performance of the trained model more robust. As mentioned, in addition or instead, contextual information $\kappa$ may include information such as any one or more of: i) the contrast agent material (Iodine, etc) that is administered, ii) its initial concentration, iii) total administered volume, iv) its administration rate, etc. In general, the contextual information $\kappa$ may describe context around the contrast agent itself.

In the following, the above-described steps, and in particular step S740 for computing the flow characteristics based on concentration values, and step S720 of computing the concentration values are described in more detail in various embodiments and sub-steps.

Turning first in more detail to step S720, precise extraction of contrast agent mass is facilitated because the specific material (such as Iodine) of contrast agent being known, and thanks to the spectral projection image as processed herein. The spectral projection image was obtained by spectral processing using for example material decomposition as mentioned before. The spectral projection image is preferably a contrast-agent-only image where contrast is solely or largely due to the contrast agent present in the field of view of the spectral imager IA1. Attenuation contributions from intervening structures, and in particular from the surrounding blood into which the contrast agent was administered, are largely eliminated thanks to spectral imaging. Thus, spectral image data as proposed herein as input allows more precisely identifying the total mass of contrast agent in the ROI, as compared to conventional X-ray projection imagery acquired in a mere energy-integrating imaging setup. In step 1120, contrast agent mass $M_I$ extraction may be done by threshold-based segmentation or other. Extraction from a given frame of total (eg iodine) mass $M_I$, may be done for a selected vessel segment, e.g. the section at the beginning of the IVUS pullback. In addition, in step S720, the volume of the lumen is obtained based on the 3D information $d$ such as the IVUS image data. A ratio $M_I/V_V$ is formed between extracted mass $M_I$ and obtained lumen volume $V_V$ to quantify the contrast agent concentration $C_I$.

If a machine learning model is used in step S720, such explicit mass extraction and lumen volume determination operations are not required, as the contrast agent concentration C can be obtained end-to-end by the trained model M. The model M processes in step S720 the input data $(\lambda,d)$ jointly, for example by passing the combined data $(\lambda,d)$ through a neural network to obtain at its output layer the said quantification $C_I$ for the sought after contrast agent concentration.

Turning now to step S740 in more detail, this may include computing based on the contrast agent/Iodine concentration any one of more of volumetric flow, bolus propagation time $t_B$, collateral flow and (inhomogeneous) mixing behavior.

For example, using the original/initial Iodine concentration $C_O$ the blood to contrast agent mixing ratio

$$R_{BI} = \frac{C_O}{C_I}$$

is calculated. This allows the determination of volumetric flow at the contrast injection site $F_O = F_I \cdot R_{BI}$, where $F_I$ is the contrast agent injection rate.

Using a second vessel segment along the IVUS pullback path (or from a second IVUS acquisition), another local concentration $C_I'$ is calculated (see above Fig. 4 for an illustration). By comparing the time curves $C_I'(t)$ and $C_I$ (t), the bolus propagation time $t_B$ between the two vessel segments is calculated $C_I'(t) = C_I(t + t_B)$.

Collateral flow to a specific vessel segment and inhomogeneous mixing behavior in aneurisms can be evaluated and highlighted by observed changes in the stable Iodine concentration $C_I$.

In embodiments, TAG (transluminal attenuation gradient) is determined in step S740 based on concentration values $C_I$. TAG may be determined using average contrast opacification in a series of regions of interest along vessel of interest. The concentration values may be used to estimate this opacification. For example, the ROIs may be evenly spaced, perpendicularly to centerline of the vessel, such as of a coronary artery. TAG procedure may be configured to measure the gradient (rate of fall-off) of contrast opacification along the vessel. For example, TAG may be defined as an average of opacifications and how this average changes along vessel. For example, TAG may be computed as regression coefficients of a line fitting a plot of average such contrast opacification versus distance from a reference point, such as the coronary ostium for example. Such TAG type processing is described by A J Einstein in Journal of the American College of Cardiology, vol. 61, issue 12, 2013, pp 1280-1282.

Various refinements of the method in Fig. 6 are envisaged herein which will now be explained.

In step S740, for determining the blood-contrast agent mixing ratio $R_{BI}$, it may be possible to derive the initial Iodine concentration Co, and/or the contrast agent injection rate (for the volumetric flow $F_O$) from spectral X-ray projection images of a guide catheter TP during the contrast agent administration. For this purpose, a segmentation of the guide catheter and a region of interest around the guide catheter may be obtained such as in a frame where the nozzle portion TP (see lower left of Fig. 2) appears in the FOV. A known model or specification of the guide catheter (any one or more of size, (3D) shape and material) improves the calculation of the initial Iodine concentration. The specification may be supplied through user interface UI. The region of interest around the guide catheter is used to monitor the injection rate over time. Thus, attenuation contributions of the guide catheter can be eliminated, and as shape/size of the guide catheter at the region of interest is known, a precise estimate of the initial contrast agent concentration as per the bolus that passed through the guide catheter may be obtained. The so obtained initial concentration based on the catheter/tube portion in a field-of-view may be used in combination with the local concentration in different ROI of the vessel interest as some diagnostic quantities may require for their computation the said initial concentration. Specifications on the shape, material of the catheter/delivery tube may be supplied by used through input interface UI or are accessed through a database, etc. The predictor component PC may not need any machine learning for determining the said initial CA contrast agent concentration based on this prior knowledge of shape/material of the catheter/delivery tube. In a similar manner, the rate of administration can be determined if time is taken into account.

In a further refinement, extracted flow rates may be combined with an additional vessel segmentation, or with the 3D IVUS model and the guide catheter pressure data to create a fluid-dynamics-based estimation of distal pressure in the vessel. This allows disease quantification indirectly for the microvasculature by calculating e.g. an IMR index. This is of particular interest for patients with no apparent epicardial vessel disease (NOCAD patients).

For the volumetric flow and/or the bolus propagation time, with the IVUS pullback across a lesion ST (such as stenosis / calcified plaque / stent / stent with restenosis). the severity or flow relevance of the vessel structure CN can be assessed.

Reference is now made to Fig. 8, which shows a flow chart of a method of training the machine learning model M, for example for use in the method of Fig. 6.

At step S810 training data is received, for example in the form of pairs $(x_k, y_k)$. Each pair includes the training input $x_k$ and the associated target $y_k$. $x_k$, as defined above.

At step S820, the training input $x_k$ is applied to an initialized machine learning model M to produce a training output.

A deviation, or residue, of the training output $M(x_k)$ from the associated target $y_k$ is quantified by a cost function F at step S830. One or more parameters of the model are adapted at step S840 in one or more iterations in an inner loop to improve the cost function. For instance, the model parameters are adapted to decrease residues as measured by the cost function. The parameters include in particular weights of the convolutional operators, in case a convolutional model M is used.

The training method then returns in an outer loop to step S810 where the next pair of training data is fed in. In step S840, the parameters of the model are adapted so that the aggregated residues of all pairs considered are decreased, in particular minimized. The cost function quantifies the aggregated residues. Forward- backward propagation or similar gradient-based techniques may be used in the inner loop. Instead of looping over individual pairs at step S810, looping is over sets of training data pair, or batches, and these are feed in a processed at once in step S820, and S830.

Examples for gradient-based optimizations may include gradient descent, stochastic gradient. conjugate gradients, Maximum likelihood methods, EM-maximization, Gauss-Newton, and others. Approaches other than gradient-based ones are also envisaged, such as Nelder-Mead, Bayesian optimization, simulated annealing, genetic algorithms, Monte-Carlo methods, and others still.

More generally, the parameters of the model M are adjusted to improve objective function F which is either a cost function or a utility function. In embodiments, the cost function is configured to the measure the aggregated residues. In embodiments the aggregation of residues is implemented by summation over all or some residues for all pairs considered. In particular, in preferred embodiments the outer summation proceeds in batches (subset of training instances), whose summed residues are considered all at once when adjusting the parameters in the inner loop. The outer loop then proceeds to the next batch and so for until the requisite number of training data instances have been processed.

In the above ML embodiments, the model M may be trained to regress or classify directly for the flow related diagnostic quantities. In such embodiments, the concentration values is implicitly computed, rather than being explicitly computed as in the above embodiments for passing to the flow assessment component where the diagnostic quantity is computed in a down-stream step analytically based on the predicted concentration. However, such explicitly predicting the sought after concentration is preferred herein.

The components of system SYS may be implemented as one or more software modules, run on one or more general-purpose processing units PU such as a workstation associated with the imager IA, or on a server computer associated with a group of imagers. The workstation or other data processing unit(s) PU may be part of the imaging apparatus IA.

Alternatively, some or all components of system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system SYS. In a further embodiment still, system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

## Claims

1.  System (SYS) for image processing, comprising:

    at least one input interface (IN) configured to receive input data comprising i) spectral projection imagery of a region of interest including a conduit (CN) for passage of a liquid (CA), the spectral projection imagery obtainable in an imaging procedure by a spectral X-ray imagining apparatus (IA1) with contrast agent (CA) present in the liquid, and ii) additional image data acquirable by a further imaging apparatus (IA2) of the non-ionizing type, the said additional image data representative of 3D information of the conduit; and
    a predictor component (PC) configured to predict, when the system is in use, based on the spectral projection imagery and the additional image data, at least a concentration of contrast agent in the said conduit.

2.  System of claim 1, wherein the 3D information is presentative of a lumen (L) of the conduit (CN).

**3.** System of claim 1 or 2, the system further comprising a flow assessment component (FAC) configured to compute a medical quantity of interest based at least on the predicted concentration of contrast agent in the said conduit, the quantity of interest descriptive of one or more flow characteristics of the liquid.

**4.** System of claim 3, wherein the said medical quantity of interest includes any one of more of: volumetric flow, propagation time of contrast agent through at least a section of said conduit, liquid - contrast agent mixing behavior, and transluminal attenuation gradient.

**5.** System of any one of the previous claims, wherein predictor component is configured to compute an initial concentration of the contrast agent upon entering the conduit and/or a rate of entering into the conduit of the said contrast agent.

**6.** System of any one of the previous claims, wherein the conduit is part of a vascular, urinary or lymphatic system of mammal, such as a human patient.

**7.** System of any one of the previous claims, wherein the further imaging apparatus is configured for intra-vascular imaging.

**8.** System of any one of the previous claims, wherein the further imaging apparatus (IA2) is any one or more of: i) an ultrasound imaging apparatus or ii) an optical tomographic coherence imaging apparatus.

**9.** System of any one of the previous claims, wherein the computed concentration of contrast agent and/or the quantity of interest is provided for processing, wherein the processing includes any one or more of: i) displaying on a display device an indication of the concentration of contrast agent and/or of the quantity of interest, ii) storing same in a medical data storage, and iii) controlling operation of medical equipment, iv) processing by decision support system (DS).

**10.** System of any one of previous claims, the predictor component (PC) based on a machine learning model (M).

**11.** An imaging arrangement (MIA) comprising the system of any one of the previous claims, further including the spectral X-ray imagining apparatus and the second imaging apparatus (IA2).

**12.** Method of image processing, comprising:

   receiving (S710) input data comprising i) spectral projection imagery of a region of interest including a conduit (CN) for passage of a liquid (CA), the spectral projection imagery obtainable in an imaging procedure by a spectral X-ray imagining apparatus (IA1) with contrast agent (CA) present in the liquid, and ii) additional image data acquirable by a further imaging apparatus (IA2) of the non-ionizing type, the said additional image data representative of 3D information of the conduit; and
   predicting (S720), when the system is in use, based on the spectral projection imagery and the additional image data, at least a concentration of contrast agent in the said conduit.

**13.** Method of training the model as per claim 10 based on training data.

**14.** A computer program element, which, when being executed by at least one processing unit, is adapted to cause the processing unit to perform the method as per claim 12 or 13.

**15.** At least one computer readable medium having stored thereon the program element of claim 14, or having stored thereon the machine learning model as per claim 10.

**FIG. 1**

**FIG. 2**

**FIG. 3**

GD

P3

P2

P1

AC

P11

P12

$C_i$

P4

$C_i'$

P5

t

t

**FIG. 4**

M

IL    L₁              LN    OL              M(X)=C

X=(λ,d,κ)

MEM'

**FIG 5**

**FIG 6**

S705

λ

d

S710

S720

C

S730

S740

**FIG 7**

S810

S820

S830

S840

**FIG 8**

Europäisches
Patentamt

European
Patent Office

Office européen
des brevets

## EUROPEAN SEARCH REPORT

Application Number

EP 23 15 2170

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/135775 A1 (MISTRETTA CHUCK [US] ET AL) 19 May 2016 (2016-05-19)<br>* paragraph [0043] *<br>* paragraph [0075] *<br>* paragraph [0010] *<br>* paragraph [0082] *<br>* figure 1A *<br>* claim 20 *<br>----- | 1-15 | INV.<br>A61B8/06<br>A61B8/12<br>A61B8/08<br>A61B5/00<br>A61B6/00 |
| | | | TECHNICAL FIELDS SEARCHED (IPC)<br><br>A61B |

The present search report has been drawn up for all claims

| Place of search | Date of completion of the search | Examiner |
|---|---|---|
| Munich | 7 August 2023 | Ordavo, Ivan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 2170

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2023

| Patent document cited in search report | Publication date | Patent family member(s) | Publication date |
|---|---|---|---|
| US 2016135775 A1 | 19-05-2016 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2

- **A J EINSTEIN.** *Journal of the American College of Cardiology,* 2013, vol. 61 (12), 1280-1282